# EUROPEAN PATENT APPLICATION

(11) **EP 4 289 840 A1**
(43) Date of publication of application: **13.12.2023**
(21) Application number: 22461563.3
(22) Date of filing: 07.06.2022
(51) Int. Cl.: C07D 417/14, C07D 417/06

(54) **A PROCESS FOR PREPARING ISAVUCONAZONIUM SULFATE**

(71) Applicant: Zaklady Farmaceutyczne Polpharma S.A., 83-200 Starogard Gdanski (PL)
(72) Inventor: HALUSZCZUK, Adam Andrzej, 80-058 Gdansk (PL); KOSIK, Kamil, 83-200 Starogard Gdanski (PL); GRAUZENIS, Maciej, 83-200 Starogard Gdanski (PL); BRODA, Ewa, 83-200 Starogard Gdanski (PL)

(57) **Abstract**

The present invention relates to a process for preparing isavuconazonium sulfate and a process for isolation and/or purification of isavuconazonium sulfate. Further, the invention relates to new isavuconazonium salts useful in the preparation and/or purification of isavuconazonium sulfate and a process for their preparation. Additionally, the invention provides a new intermediate useful in the synthesis of isavuconazonium.

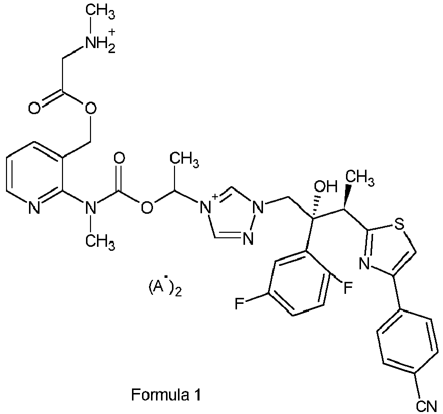

## Description

### Background of the invention

The present invention relates to a process for preparing isavuconazonium sulfate and a process for isolation and/or purification of isavuconazonium sulfate. Further, the invention relates to new isavuconazonium salts useful in the preparation and/or purification of isavuconazonium sulfate and a process for their preparation. Additionally, the invention provides a new intermediate useful in the synthesis of isavuconazonium.

Isavuconazonium is named 1-[[N-methyl-N-3-[(methylamino)acetoxymethyl]pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium and has the following structure:

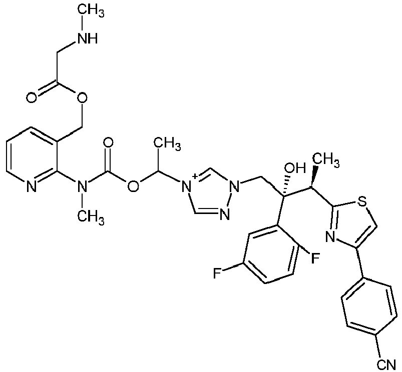

Isavuconazonium is a water-soluble prodrug form of isavuconazole with broad-spectrum antifungal activity. It is marketed under the trade name Cresemba^{®} as a sulfate salt and is indicated for the treatment of invasive aspergillosis and mucormycosis. Cresemba^{®} is prescribed as an oral dosage form as well as a powder for intravenous application.

Isavuconazonium sulfate, 1-[[N-methyl-N-3-[(methylamino)acetoxymethyl]pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium sulfate, has the following structure: 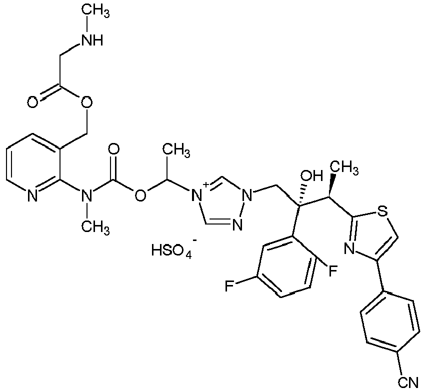 and is disclosed in WO2001032652. The application however does not specifically disclose the synthesis of isavuconazonium sulfate, it discloses a process for preparation of isavuconazonium dihydrochloride with the use of chromatographic purification, a strong anion exchange resin and lyophilization of the final product.

A different approach to the synthesis of isavuconazonium salts is disclosed in WO2021037597 and is based on the reaction of compound (2) with a first acid HA₁ to obtain compound (3) 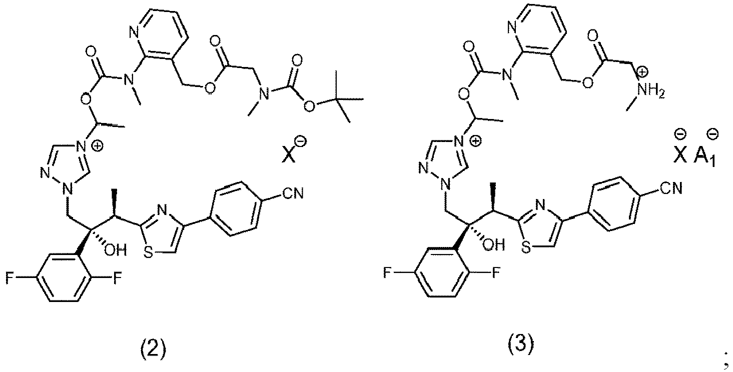 wherein X means Cl or Br, or I, and then reacting compound of formula (3) with a second acid HA₂ in a water immiscible solvent to obtain a mixture comprising compound (4) in the water immiscible solvent:

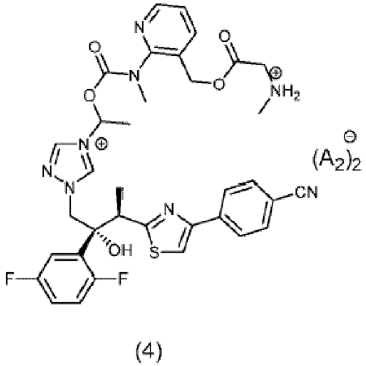

The description explicitly mentions only acids of the formula CX₃(CX₂)mCOOH, specifically CF₃COOH, as HA₂. Further, compound (4) can be converted to isavuconazonium sulfate in two steps: by reacting it with sulfuric acid in a water immiscible solvent and precipitation of the product (9) followed by reacting the precipitated product (9) with Ba²⁺. Isavuconazonium sulfate is obtained as an aqueous solution, no isolation methods of the product are described.

WO2021129580 discloses a method for preparing isavuconazonium sulfate involving obtaining a trifluoroacetate salt of isavuconazonium and converting it to the sulfate salt in the presence of a compound having a bisulfate ion. The workup includes the use of microporous resins and lyophilization of isavuconazonium sulfate from the aqueous phase.

The idea of purification of isavuconazonium using various salts as intermediates instead of using an ion exchange resin is not new and appears to be the right direction towards simplifying and reducing costs of the overall process.

However, the prior art's process, where a salt of isavuconazonium, in particular isavuconazonium trifluoroacetate is used as an intermediate to obtain isavuconazonium sulfate, may reveal certain drawbacks.

In particular, WO2021037597discloses a process, where isavuconazonium bisulfate (compound (9)) is obtained from the trifluoroacetate salt of isavuconazonium by precipitation in isopropyl acetate and reaction of the precipitate with barium acetate. The present inventors have found that in this process co-precipitation of isavuconazonium trifluoroacetate or a mixed salt of isavuconazonium bisulfate and trifluoroacetate is inevitable, thus trifluoroacetate salt will be present in the final product isavuconazonium sulfate. Additionally acetic acid coming from barium acetate may also be present in the final product. Moreover, in WO2021037597the yield of conversion is not given and the application does not disclose any isolation methods for the final sulfate salt of isavuconazonium, consequently no characteristics of the product in terms of ion content (trifluoroacetate ions, barium ions) and solvent residues can be extracted from the disclosure. The scale of the reaction is quite small, 0.25 g.

Moreover, attempts to reproduce the process of WO2021129580 and to obtain trifluoroacetate salt of isavuconazonium (compound V in the application) of declared purity after deprotection have failed. Additionally, the yield of conversion of trifluoroacetate salt into sulfate salt of isavuconazonium with sodium bisulfate was not satisfactory due to the presence of large amounts of unwashed trifluoroacetate in water.

The present inventors have surprisingly found that for the respective stage of exchanging an anion to sulfate without the use of resins, where prior art's drawbacks would be eliminated or at least significantly reduced, not only a difference in strength of acids but also a significant difference in lipophilicity of respective acids is required.

A significant difference in lipophilicity allows to avoid an incomplete exchange of anions of the original salt by sulfate/bisulfate anions and allows to differentiate solubility of respective salts in water and organic solvents enabling isolation and reduction or elimination of co-precipitation of salts.

Unexpectedly, it has been found that the process according to the present invention provides isavuconazonium sulfate with improved yield and very high purity without the use chromatographic purification, ion exchange resins and with a minimum of isolated intermediates. The progress of ion exchange is strictly controlled in the process making it possible to achieve quantitative ion exchange rates.

### Detailed description of the invention

In one aspect the present invention relates to a process for the preparation on isavuconazonium sulfate comprising
a1) contacting the salt of Formula 1 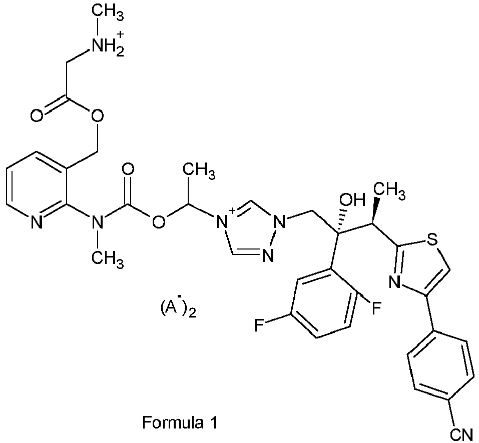 in a system of at least one organic solvent and water with a source of bisulfate anions, wherein A is an anion of a lipophilic organic acid HA.

Preferably, the lipophilic organic acid HA has a pKa value in water between -2 and 4. Suitable lipophilic organic acids are aromatic carboxylic acids, preferably aromatic monocarboxylic acids. Preferred aromatic monocarboxylic acids are selected from benzoic acid and naphthalenic acid, wherein the aromatic moiety is unsubstituted or is substituted with one or more groups selected from hydroxyl, (C₁-C₆)-alkyl-(C=O)-, halogen, (C₁-C₆)alkoxyl, nitro, cyano, (C₁-C₆)alkyl. Preferably, the lipophilic organic acid is selected from 2,3,4,5,6-pentafluorobenzoic acid and 2,6-dihydroxybenzoic acid. Most preferably the lipophilic organic acid is 2,3,4,5,6-pentafluorobenzoic acid.

The isavuconazonium salt with a lipophilic organic acid of Formula 1 is converted into a bisulfate salt by reaction with a source of bisulfate ions. The ion exchange reaction is conducted in a system of at least one organic solvent and water.

The at least one organic solvent of the above system is selected from at least one water miscible solvent, at least one water immiscible solvent, and a mixture thereof. The at least one water miscible solvent is selected from ketones, for example acetone; alcohols, for example methanol, ethanol, isopropanol, n-propanol; ethers, for example THF,1,4-dioxane; acetonitrile or acetic acid. Preferably, the water miscible solvent is THF. The at least one water immiscible solvent is selected from esters of acetic acid, for example ethyl acetate, isopropyl acetate; ethers, for example diethyl ether, diisopropyl ether, methyl tert-butyl ether; halogenated (C₁-C₂)-alkanes, for example dichloromethane, dichloroethane, chloroform; aromatic solvents, for example toluene, xylenes. Preferably, the water immiscible solvent is isopropyl acetate.

Miscibility is the property of two substances to mix in all proportions, that is, to fully dissolve in each other at any concentration to form a homogenous solution. For example, water and ethanol are miscible because they mix in all proportions. Conversely, substances are said to be immiscible if there are certain proportions in which the mixture does not form a homogenous solution. Miscibility of two substances is often determined optically, for example when the two miscible liquids are combined the resulting liquid is clear. Likewise if upon mixing two substances two phases form (for example an aqueous phase and an organic phase) the two substances are also said to be immiscible. On the other hand, if two immiscible liquids are combined, the resulting liquid is cloudy and no phase separation occurs. That is, where an organic solvent and water are mixed, the mixture is said to be immiscible when either two phases form or an emulsion is observed. In situations where emulsions form, it is known that phase formation and solvent separation may be improved by the addition of solid sodium chloride or a solution of sodium chloride.

In one embodiment an aqueous solution of sulfuric acid is used as a source of bisulfate anions. The aqueous solution of sulfuric acid is thus added to the salt of Formula 1 in the system of at least one organic solvent and water. The salt of Formula 1 is present in dissolved form. The concentration of sulfuric acid is within the range of 10-25% (w/w). The molar ratio of sulfuric acid to isavuconazonium is at least 1:1. The reaction mixture is stirred with cooling. In one variant of this embodiment where the system of at least one organic solvent and water is a biphasic system, then the phases are separated and the organic phase is discarded. In another variant of this embodiment, where the system of at least one organic solvent and water is a monophasic system, then the reaction mixture is extracted with a water immiscible solvent, and the organic phase is discarded. The ion exchange rate of lipophilic organic acid salt of isavuconazole to bisulfate is quantitative. The progress of the exchange can be controlled by the number of extractions.

In one embodiment an aqueous solution of a bisulfate salt is used as a source of bisulfate anions.

The aqueous solution of a bisulfate salt is thus added to the salt of Formula 1 in the system of an organic solvent and water. The salt of Formula 1 is present in dissolved form. The bisulfate salt is selected from alkali metal, alkaline earth metal and ammonium bisulfates. Preferably, the bisulfate salt is sodium or ammonium bisulfate. The molar ratio of the bisulfate salt to isavuconazonium is at least 1:1. The reaction mixture is stirred with cooling. In one variant of this embodiment where the system of at least one organic solvent and water is a biphasic system, then the phases are separated and the organic phase is discarded. In another variant of this embodiment, where the system of at least one organic solvent and water is a monophasic system, then the reaction mixture is extracted with a water immiscible solvent, and the organic phase is discarded. The ion exchange rate of lipophilic organic acid salt of isavuconazonium to bisulfate is quantitative.

Alternatively, bisulfate salt as a source of bisulfate anions may be added as a solid, which dissolves in the system of at least one organic solvent and water of step a1).

In one embodiment the lipophilic organic acid salt of Formula 1 is provided as a solution in a water immiscible solvent. Suitable organic solvents include ethers, for example methyl tert-butyl ether, 2-methyltetrahydrofurane, diethyl ether, diisopropyl ether; esters of acetic acid, for example methyl acetate, ethyl acetate, isopropyl acetate, butyl acetate; ketones, for example methyl ethyl ketone, methyl isobutyl ketone; halogenated (C₁-C₂)-alkanes, for example dichloromethane, dichloroethane, chloroform; aromatic solvents, for example toluene, xylenes.

In one embodiment the lipophilic organic acid salt of Formula 1 is provided as a solution in a mixture of water and water miscible solvent, such as alcohol, for example methanol, ethanol, n-propanol, isopropanol; ketone, for example acetone; ether, for example THF, 1,4-dioxane; acetonitrile or acetic acid. Preferably the water miscible solvent is THF. The ratio of water and water miscible solvent is preferably around 1:1. Next, an aqueous solution of sulfuric acid or a bisulfate salt is added. The molar ratio of sulfuric acid or bisulfate salt to isavuconazonium is at least 1:1. The reaction mixture is homogenous and is stirred with cooling. In the next step the reaction mixture is extracted with a water immiscible solvent. The water immiscible solvent can be selected from esters of acetic acid, for example ethyl acetate, isopropyl acetate; ethers, for example methyl tert-butyl ether, diisopropyl ether, diethyl ether; halogenated (C₁-C₂)-alkanes, for example dichloromethane, dichloroethane, chloroform; aromatic solvents, for example toluene, xylenes. Preferably, the water immiscible solvent is isopropyl acetate.

Isavuconazonium bisulfate obtained in step a1) is then converted to isavuconazonium sulfate.

Accordingly, the invention provides a process for preparing, isolating and purifying isavuconazonium sulfate.

The process for preparing, isolating and/or purifying isavuconazonium sulfate comprises the following steps:
(i) providing an aqueous solution of isavuconazonium bisulfate,
(ii) adding an alkali metal sulfate or ammonium sulfate, and an organic solvent,
(iii) separating the organic phase,
(iv) combining the obtained organic phase with an antisolvent or a mixture of antisolvents to precipitate isavuconazonium sulfate.

Analogous process can also be used to isolate and purify isavuconazonium sulfate starting from isavuconazonium sulfate. Accordingly, the invention provides a process for isolation and purification of isavuconazonium sulfate comprising the following steps:
(i) providing an aqueous solution of isavuconazonium sulfate,
(ii) adding an alkali metal sulfate or ammonium sulfate, and an organic solvent,
(iii) separating the organic phase,
(iv) combining the obtained organic phase with an antisolvent or a mixture of antisolvents to precipitate isavuconazonium sulfate.

In one embodiment the process for preparing, isolating and/or purifying isavuconazonium sulfate comprises the following steps:
(i) providing an aqueous solution of isavuconazonium bisulfate and/or sulfate,
(ii) adding an alkali metal sulfate or ammonium sulfate, and an organic solvent,
(iii) separating the organic phase,
(iv) combining the obtained organic phase with an antisolvent or a mixture of antisolvents to precipitate isavuconazonium sulfate.

The sulfate salt used in step (ii) of the above processes is water soluble. Preferably, an alkali metal salt, for example, sodium sulfate, potassium sulfate or ammonium sulfate is used. More preferably the sulfate salt used in step (ii) is ammonium sulfate.

This process can be performed with isavuconazonium bisulfate and/or isavuconazonium sulfate as starting material, irrespectively of the process by which these compounds were obtained. The conversion, isolation and/or purification is effected by addition of water-soluble alkali metal, for example sodium or potassium, or ammonium sulfate salt, to the aqueous solution of isavuconazonium bisulfate and/or sulfate. The advantage of using water-soluble alkali metal, for example sodium or potassium, or ammonium sulfate salt is that it simultaneously buffers isavuconazonium bisulfate to sulfate and saturates the aqueous phase.

The aqueous solution of isavuconazonium bisulfate can be either directly taken from step a1) without further operations or it can be prepared, for example, by dissolving the solid compound 9 as obtained in WO2021037597 in water. Then water-soluble alkali metal, for example sodium or potassium, or ammonium sulfate salt and a suitable organic solvent are added.

The molar ratio of the water-soluble alkali metal, for example sodium or potassium, or ammonium sulfate salt and isavuconazonium bisulfate and/or sulfate is within the range of from 1:1 to 50:1. Preferably the molar ratio is within a range from 10:1 to 20:1.

The suitable organic solvent of step (ii) can be a water miscible or a water immiscible solvent, or a mixture thereof. The organic solvent of step (ii) is selected from ethers, for example THF, 2-methyltetrahydrofurane; esters of acetic acid, for example isopropyl acetate; ketones, for example acetone, methyl ethyl ketone; alcohols, for example methanol, isopropanol; halogenated (C₁-C₂)-alkanes, for example dichloromethane; acetonitrile or acetic acid; or mixtures thereof. Preferably the organic solvent is THF.

Then, in step (iii) of the above processes, the phases are separated and the aqueous phase is discarded. The organic phase can be repeatedly washed with an aqueous solution of water-soluble alkali metal, for example sodium or potassium, or ammonium sulfate salt.. The organic phase in step (iv) of the above processes is combined with an antisolvent or a mixture of polar solvent with antisolvent, and isavuconazonium sulfate is precipitated.

The antisolvent for step (iv) of the above processes is selected from non-polar aprotic solvents like ethers, for example diethyl ether, methyl tert-butyl ether; alkanes, for example pentane, hexane, cyclohexane, heptane. Polar solvents comprise for example acetone, isopropanol, acetonitrile. Preferably, the organic phase is step (iv) of the above processes is combined with a mixture of methyl tert-butyl ether and isopropanol.

The filtered and dried crude isavuconazonium sulfate being a product of the above processes contains approximately up to 10% of residual solvents. The isolated product has very high ionic and chemical purity, the sulfate ions content is close to theoretical value, purity as measured by HPLC is above 98%. Due to the fact that the isolation takes place from an organic medium and not water, the product can be dried from residual solvents under very mild conditions hence no decomposition of the product takes place.

The following steps have the purpose to further reduce the sodium or potassium or ammonium ion content and residual solvent content in the final product, if needed.

Crude isavuconazonium sulfate is dissolved in a first solvent or a mixture of a first and a second solvent, wherein the first solvent is a solvent in which isavuconazonium sulfate has good solubility and the second solvent is an antisolvent. Suitable first solvent includes methanol and acetic acid. Suitable second solvent includes ketones, for example acetone; ethers, for example THF, 2-methyltetrahydrofurane; alcohols, for example ethanol, isopropanol, n-propanol; acetonitrile. Preferably, the first solvent is methanol and the second solvent is acetone.

The obtained solution is filtered off and combined with an antisolvent or a mixture of at least two antisolvents. Suitable antisolvents include ketones, for example acetone; ethers, for example methyl tert-butyl ether, diisopropyl ether, diethyl ether; esters of acetic acid, for example ethyl acetate, isopropyl acetate; alkanes, for example pentane, hexane, heptane, cyclohexane; aromatic solvents, for example toluene, xylenes. Preferably the antisolvent is methyl tert-butyl ether or a mixture of antisolvents comprises acetone and methyl tert-butyl ether.

Isavuconazonium sulfate precipitates from the combined mixture, it is filtered off and the precipitate is washed with cyclohexane. The washing with cyclohexane allows to remove residual solvents contained in the precipitated product and replace them for cyclohexane, which can be easily removed during lyophilization. Then the product is lyophilized under very mild conditions. The final isavuconazonium sulfate is characterized by high HPLC purity of above 98%, and very high ionic purity.

The invention also provides 4-{2-[(1R,2R)-(2,5-difluorophenyl)-2-hydroxy-1-methyl-3-(1H-1,2,4-triazol-1-yl)propyl]-1,3-thiazol-4-yl}benzonitrile hydrobromide THF solvate (isavuconazole hydrobromide THF solvate, ISV-3 HBr THF) and a process for its preparation.

The synthesis of isavuconazonium sulfate according to the present disclosure begins with obtaining 4-{2-[(1R,2R)-(2,5-difluorophenyl)-2-hydroxy-1-methyl-3-(1H-1,2,4-triazol-1-yl)propyl]-1,3-thiazol-4-yl}benzonitrile (ISV-3) by reacting (2R,3R)-3-(2,5-difluorophenyl)-3-hydroxy-2-methyl-4-(1,2,4-triazol-1-yl)butanethioamide (ISV-1) with 2-bromo-4'-cyanoacetophenone (ISV-2) in ethanol. After removing ethanol by distillation under reduced pressure, the obtained residue is dissolved in THF, optionally with a small addition of ethanol and ISV-3 HBr THF solvate precipitates from the solution upon cooling.

Advantageously, it has been found that isavuconazole hydrobromide THF solvate is solid. Preferably, it is crystalline. Most preferably it displays the following characterizing peaks in XRPD:
XRPD 2θ (°) ± 0.2°: 5.8, 8.3, 11.6, 13.6, 15.8, 16.6, 19.0, 19.7, 22.3, 26.2

It has also been found that isavuconazole hydrobromide THF solvate demonstrates improved stability and high chemical purity in comparison to isavuconazole hydrobromide disclosed in the prior art, moreover it is easy to handle on both small- and large-scale.

The content of HBr and THF in the isavuconazole hydrobromide THF solvate is as listed below:

| | | |
|---|---|---|
| HBr | theoretical | 13,4% w/w, |
| | experimental | 13,2% w/w, |
| THF | theoretical | 12,2% w/w, |
| | experimental | 12,2% w/w. |

In the next step, isavuconazole hydrobromide THF solvate is suspended in dichloromethane and an aqueous solution of NaHCO₃ is added. The phases are separated and the organic phase is washed with brine. Subsequently, the solvents are distilled off under reduced pressure and isavuconazole free base is obtained (ISV-3).

Subsequently, isavuconazole free base ISV-3 is reacted with N-methyl N-(3-[((N-tert-butoxycarbonyl-N-methylamino)acetoxy)methyl]pyridin-2-yl)carbamic acid (1-chloroethyl) ester (ISV-4) in acetonitrile to obtain 1-[[N-methyl-N-3-[(t-butoxycarbonylmethylamino)acetoxymethyl] pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride/bromide (ISV-5 CI/Br). The Boc group in ISV-4 can be substituted by any nitrogen protective group that can be removed under acidic conditions.

The next step comprises deprotection of the product ISV-5 Cl/Br with the use of a hydrohalic acid HX, wherein X is a halogen atom selected from Cl, Br and I.

The invention also provides a process for the preparation of the compound of Formula 1, as defined above, wherein
a0) the compound of Formula 2 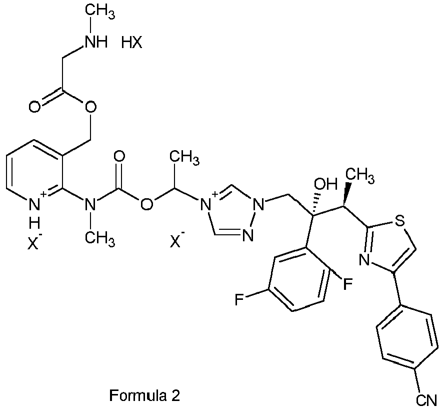 is contacted with a salt of a lipophilic organic acid HA,
wherein X is a halogen atom X selected from Cl, Br and I.

Preferably, the compound of Formula 2 which is 1-{(2R,3R)-3-[4-(4-cyanophenyl)-1,3-thiazol-2-yl]-2-(2,5-difluorophenyl)-2-hydroxybutyl}-4-[(1RS)-1-({methyl[3-({[(methylamino)acetyl]oxy}methyl)pyridin-2-yl]carbamoyl}oxy)ethyl]-1H-1,2,4-triazol-4-ium halide hydrogenhalide, wherein halogen atom X in the Formula 2 can be selected from Cl, Br and I, is dissolved in water, an organic solvent is added thereto, and the resulting system is contacted with a salt of lipophilic organic acid HA.

Preferably, the lipophilic organic acid HA has a pKa value in water between -2 and 4. Suitable lipophilic organic acids are aromatic carboxylic acids, preferably aromatic monocarboxylic acids. Preferred aromatic monocarboxylic acids are selected from benzoic acid and naphthalenic acid, wherein the aromatic moiety is unsubstituted or is substituted with one or more groups selected from hydroxyl, (C₁-C₆)-alkyl-(C=O)-, halogen, (C₁-C₆)-alkoxyl, nitro, cyano, (C₁-C₆)-alkyl. Preferably, the lipophilic organic acid is selected from 2,3,4,5,6-pentafluorobenzoic acid and 2,6-dihydroxybenzoic acid. Most preferably the lipophilic organic acid is 2,3,4,5,6-pentafluorobenzoic acid.

Preferably the salt of the lipophilic organic acid HA is a water-soluble salt with alkali metal, alkaline earth metal or ammonia and amine. Preferably, the salt used in step a0) is sodium 2,3,4,5,6-pentafluorobenzoate, ammonium 2,3,4,5,6-pentafluorobenzoate or calcium 2,3,4,5,6-pentafluorobenzoate. More preferably, the salt is sodium 2,3,4,5,6-pentafluorobenzoate. The salt can be dissolved in water and contacted with a solution of isavuconazonium halide hydrogenhalide of Formula 2.

After adding the salt of lipophilic organic acid HA and an organic solvent to the aqueous solution of isavuconazonium halide hydrogenhalide of Formula 2, the reaction mixture is stirred, the two phases are separated and the aqueous phase is discarded.

The organic solvent in step a0) is selected from ethers, for example methyl tert-butyl ether, 2-methyltetrahydrofurane, diethyl ether, diisopropyl ether; esters of acetic acid, for example methyl acetate, ethyl acetate, isopropyl acetate, butyl acetate; ketones, for example methyl ethyl ketone, methyl isobutyl ketone; halogenated (C₁-C₂)-alkanes, for example dichloromethane, dichloroethane, chloroform; aromatic solvents, for example toluene, xylenes.

The organic phase can be contacted with another portion of the aqueous solution of lipophilic organic acid salt until all anions are exchanged. The product, salt of isavuconazonium with a lipophilic organic acid HA of Formula 1, is present in the organic phase due to its limited solubility in water and lipophilic properties. The phase separation allows for simple removal of inorganic compounds.

The organic phase can be used without further workup in step a1). Alternatively, the solvents can be distilled off under reduced pressure and the residue can be dissolved in a mixture of water miscible solvent and water and the solution can be used for step a1).

Further, the invention relates to a salt of isavuconazonium with a lipophilic organic acid HA of Formula 1.

The lipophilic organic acid HA has a pKa in water between -2 and 4 and is preferably selected from aromatic carboxylic acids, more preferably aromatic monocarboxylic acids.

More preferably, the aromatic monocarboxylic acid is selected from benzoic acid and naphthalenic acid, wherein the aromatic moiety is unsubstituted or is substituted with one or more groups selected from hydroxyl, (C₁-C₆)-alkyl-(C=O)-, halogen, (C₁-C₆)-alkoxyl, nitro, cyano, (C₁-C₆)-alkyl.

In a preferred embodiment the salt of isavuconazonium of Formula 1 is the salt with the lipophilic organic acid HA which is 2,3,4,5,6-pentafluorobenzoic acid or 2,6-dihydroxybenzoic acid, most preferably 2,3,4,5,6-pentafluorobenzoic acid.

The present invention relates also to a use of a salt of Formula 1, as defined above, in the preparation of isavuconazonium sulfate.

The following examples describe the present invention in detail but are not to be construed to limit the present invention.

### Analytical methods

XRPD: The X-Ray diffractograms were recorded on a Rigaku MiniFlex 600 X-Ray Diffractometer (Bragg-Brentano geometry) with Cu-Kα radiation (1.5406 Å) within the angle range of 2 to 40° 2θ with 0.01° step and scan speed of 6.0 deg/min. Limiting slit was adjusted to 10 mm and Kβ filter was during the entire experiment.

### Examples

### Example 1: Synthesis of : 4-{2-[(1R,2R)-(2,5-difluorophenyl)-2-hydroxy-1-methyl-3-(1H-1,2,4-triazol-1-yl)propyl]-1,3-thiazol-4-yl}benzonitrile hydrobromide THF solvate (ISV-3 HBr THF)

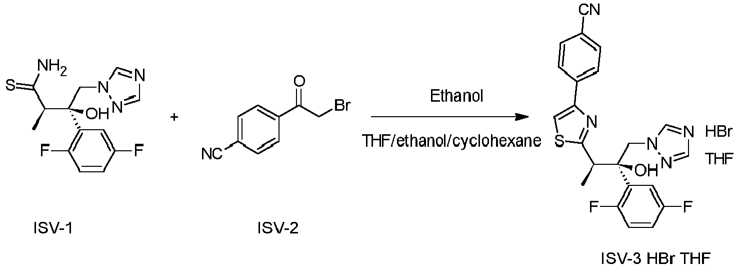

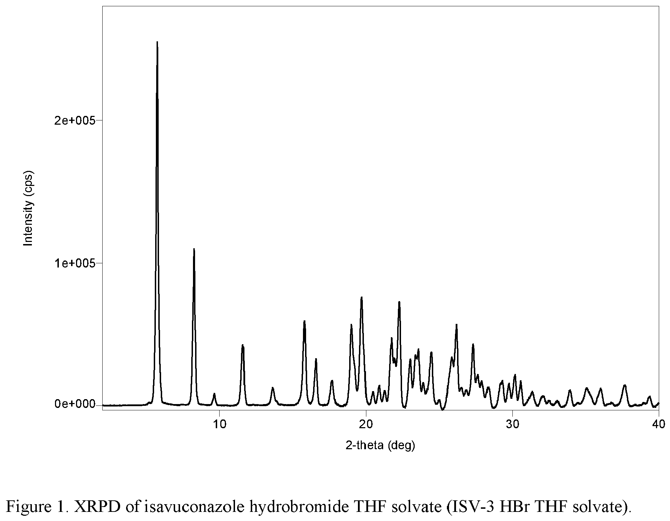

38 g of (2R,3R)-3-(2,5-difluorophenyl)-3-hydroxy-2-methyl-4-(1,2,4-triazol-1-yl)butanethioamide (ISV-1) (0.122 mol), 27.74 g of 2-bromo-4'-cyanoacetophenone (ISV-2) (0.124 mol) are placed in a 1 l glass reactor with an anchor stirrer together with 280 ml of ethanol denatured with acetone. The reaction mixture is heated to reflux and stirred at reflux for 1.5 h. Then it is cooled to about 50 - 60°C and ethanol is removed by distillation under reduced pressure. The residue is dissolved in 266 ml of THF and 9.5 ml of ethanol denatured with acetone at 50 - 60°C. The reaction is stirred for 30 minutes at 50 - 60°C, then cooled to 10 - 15°C. 150 ml of cyclohexane are added dropwise. The reaction is cooled to 0 - 5°C, stirred at 0 - 5°C for 4-6 h and the resulting precipitate is filtered off. The solid product is washed twice with a mixture THF:cyclohexane (90 ml of THF and 90 ml of cyclohexane for each washing). The product is dried at 30 - 40°C under vacuum to yield 54.5 g (76%, 0.0925 mol) of 4-{2-[(1R,2R)-(2,5-difluorophenyl)-2-hydroxy-1-methyl-3 -(1H-1,2,4-triazol-1-yl)propyl]-1,3-thiazol-4-yl}benzonitrile hydrobromide THF solvate (ISV-3 HBr THF) (HPLC purity 99.8-99.9%). XRPD of the product is presented in Figure 1.

### Example 2: Synthesis of 1-[[N-methyl-N-3-[(t-butoxycarbonylmethylamino)-acetoxymethyl[pyridin-2-yl[carbamoyloxy[ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride/bromide (ISV-5 Cl/Br)

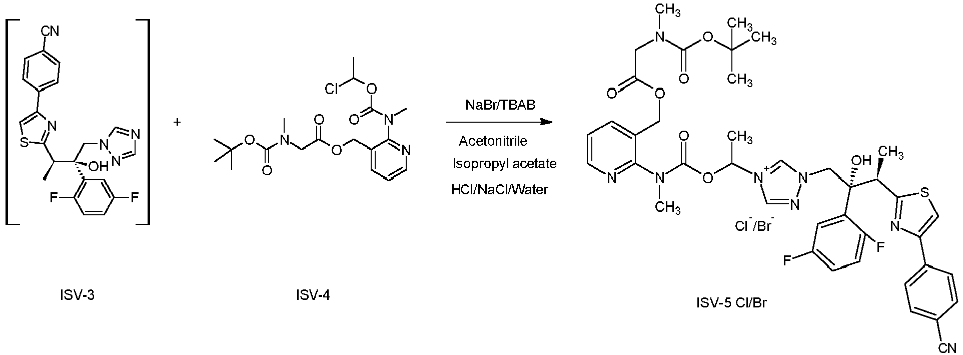

51 g (0.0866 mol) of ISV-3 HBr THF solvate is suspended in 180 ml dichloromethane and 192 ml of 5% (w/w) aqueous solution of NaHCO₃ is added. The reaction mass is stirred at 20 - 25°C for 30 minutes. Phases are separated. The organic phase is washed with 110 ml of 20% brine. Next, solvents are distilled off from the organic phase under reduced pressure till dryness. The obtained ISV-3 as an oil/foam is used in next step. ISV-3 residue is dissolved in 77 ml of acetonitrile at 30 - 40°C. Then the reaction mixture is cooled to 20 - 30°C. N-methyl N-(3-[((N-tert-butoxycarbonyl-N-methylamino)acetoxy)methyl]pyridin-2-yl)carbamic acid (1-chloroethyl) ester (ISV-4) (45.7 g, 0.11 mol) is dissolved in 51 ml of acetonitrile and added to the ISV-3 solution. 15.94 g (0.155 mol) of milled NaBr and 10.58 g (0.0328 mol) of TBAB are added. The reaction is heated to 48 - 55°C and stirred at this temperature for 16 h. 6.4 ml of DM water is added and the reaction is stirred for additional 2 h at 48 - 55°C. Then inorganic solids are filtered off and the filter is washed with 40 ml of acetonitrile. The combined filtrates are distilled till dryness. 330 ml of isopropyl acetate and 200 ml of DM water are added to the residue. The reaction is stirred for 30-60 minutes at 25 - 35°C. Then phases are separated and the water phase is discarded. The organic phase is washed three times with 200 ml of an aqueous solution of NaCl and HCl (5.5% NaCl, 0.2% HCl) at 20 - 30°C. The organic phase is subsequently washed with 200 ml of 2% (w/w) aqueous solution of NaCl and solvents are distilled of under reduced pressure. The residue is dissolved in 200 ml of dichloromethane and the solvent is distilled off under reduced pressure. Then, the residue is again dissolved in 200 ml of dichloromethane and the solvent is distilled off under reduced pressure. The residue is dissolved in 70 ml of dichloromethane and 76.5 ml of cyclohexane at 25 - 35°C. The obtained solution is added dropwise to 760 ml of cyclohexane at 10 - 20°C. The dropping funnel is washed with a mixture of 6.5 ml of dichloromethane and 6.5 ml of cyclohexane. The reaction mixture with the precipitated product is stirred for 30 minutes at 10 - 20°C. The product is filtered off, washed with 190 ml of cyclohexane and subsequently dried at 30 - 40°C under vacuum. 95 g (containing about 25% of residual solvents, yield about 90%, HPLC purity about 95%) of ISV-5 Cl/Br (counter ion containing about 80% of chlorides and 20% of bromides) is obtained.

### Example 3: Synthesis of 1-1(2R,3R)-3-[4-(4-cyanophenyl)-1,3-thiazol-2-yl]-2-(2,5-difluorophenyl)-2-hydroxybutyl}-4-[(1RS)-1-({methyl[3-({[(methylamino)acetyl]oxyl-methyl)pyridin-2-yl]carbamoyl}oxy)ethyl]-1H-1,2,4-triazol-4-ium bromide hydrobromide (isavuconazonium bromide hydrobromide, ISV Br HBr)

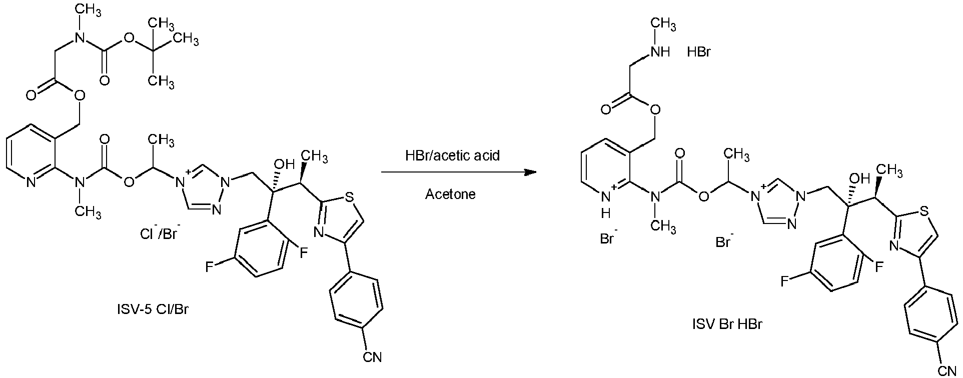

40 g (about 0.035 mol) of ISV-5 Cl/Br is dissolved in 480 ml of acetone at 20 - 25°C and 40 ml of 33% solution of HBr (0.22 mol) in acetic acid are added dropwise. The reaction is stirred for 90 minutes at 20 - 25°C, solid product is filtered off and washed with 2 x 60 ml of acetone. The product is purged/dried by nitrogen flow on the filter. The obtained isavuconazonium bromide hydrobromide is used in the next step without any additional drying.

### Example 4a: Synthesis of isavuconazonium sulfate

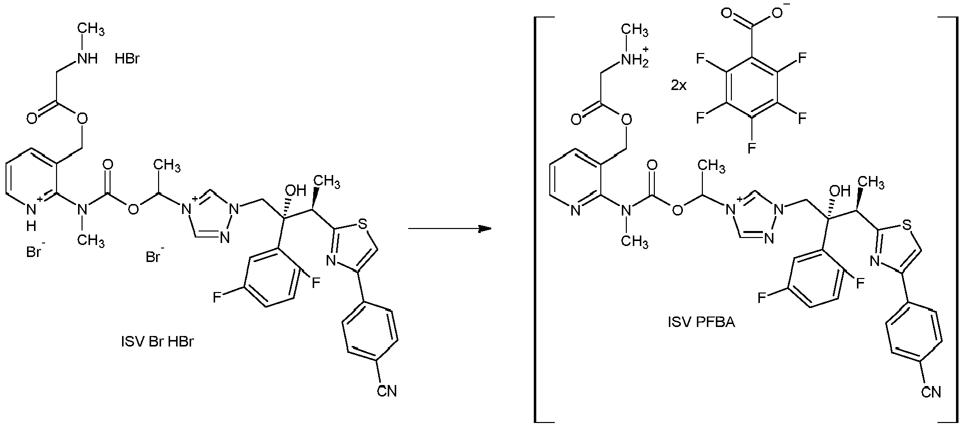

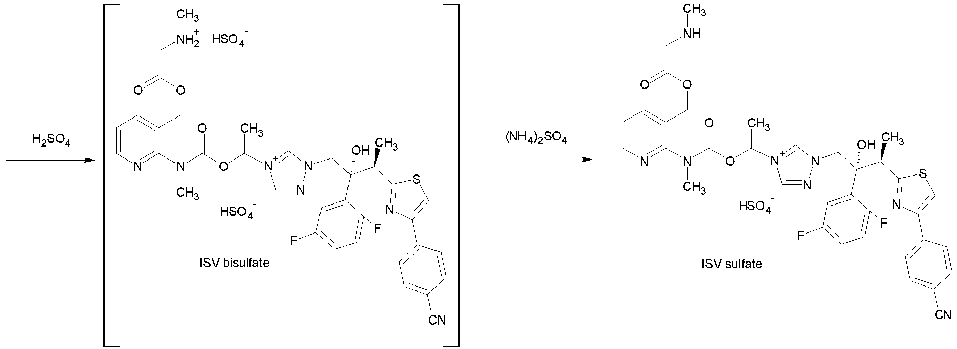

Isavuconazonium bromide hydrobromide (ISV Br HBr) obtained as in Example 3 is dissolved in 40 ml of DM water and 220 ml of isopropyl acetate at 0 - 5°C. A solution of 10 g (0.25 mol) of NaOH in 330 ml of water is prepared in a second reactor. 55 g (0.259 mol) of 2,3,4,5,6-pentafluorobenzoic acid is added to the NaOH solution, stirred till dissolution and cooled to 0 - 10°C. 2/3 of the prepared sodium 2,3,4,5,6-pentafluorobenzoate solution are added dropwise to the ISV Br HBr solution and the obtained mixture is stirred for 20 minutes at -5 - +5°C. The phases are separated and the aqueous phase is discarded. The rest (1/3) of the sodium 2,3,4,5,6-pentafluorobenzoate solution is added to the organic phase and the obtained mixture is stirred for 20 minutes at -5 - +10°C. 100 ml of DM water is added to the organic phase. Then, 30 ml of 25% (w/w) aqueous solution of sulfuric acid are added. The reaction mixture is stirred for 20 minutes at -5 - +5°C. The phases are separated and the organic phase is discarded. 1.5 g of ammonium sulfate is added to the aqueous phase and stirred till dissolution. The aqueous phase is washed with 2 x 120 ml of isopropyl acetate at -5 - +10°C. The product is isavuconazonium bisulfate contained in the aqueous phase.

### Example 4b: Isolation of crude isavuconazonium sulfate

65 g of ammonium sulfate are added to the aqueous solution of Example 4a and stirred for 10 minutes at -5 - +10°C. 40 ml of THF are added and the obtained mixture is stirred for 20 minutes at -5 - +10°C. The phases are separated and the aqueous phase is discarded. The organic phase is washed twice with 65 ml of 40% (w/w) aqueous solution of ammonium sulfate. 6 ml of methanol, 48 ml of isopropanol and 6 g of anhydrous magnesium sulfate are added to the organic phase. The obtained suspension is stirred for 20 minutes at -5 - +10°C. Filtration is performed to remove inorganic solids. The filter is washed with a mixture of 9 ml of THF and 1 ml of methanol. In a second reactor 550 ml of methyl tert-butyl ether and 95 ml of isopropanol are cooled to 0 - 5°C. The filtrate is added dropwise to a mixture of methyl tert-butyl ether and isopropanol. The obtained suspension is stirred at 0 - +10°C for 30 minutes and the precipitated product is filtered off. The product on the filter is washed with 2 x 60 ml of methyl tert-butyl ether and dried at RT (20 - 25°C) under vacuum. 25 g of isavuconazonium sulfate crude are obtained (containing about 10% of solvents, 0.027 mol, yield about 77%).

### Example 5a: Synthesis of izavuconazonium sulfate

ISV Br HBr as a solution in dichloromethane obtained as in Example 3 is dissolved in 40 ml of DM water and 120 ml of dichloromethane at 0 - 5°C. A solution of 10 g (0.25 mol) of NaOH in 330 ml of water is prepared in a second reactor. 55 g (0.259 mol) of 2,3,4,5,6-pentafluorobenzoic acid is added to the NaOH solution, stirred till dissolution and cooled to 0 - 10°C. 2/3 of prepared sodium 2,3,4,5,6-pentafluorobenzoate solution are added to the ISV Br HBr solution and the obtained mixture is stirred for 20 minutes at -5 - +10°C. The phases are separated and the aqueous phase is discarded. The rest (1/3) of the sodium 2,3,4,5,6-pentafluorobenzoate solution is added to the organic phase and the obtained mixture is stirred for 20 minutes at -5 - +10°C. Phases are separated. Isavuconazonium 2,3,4,5,6-pentafluorobenzoate is obtained in dichloromethane. The solvents are distilled off under reduced pressure. The residue is dissolved in 40 ml of THF and 30 ml of water. Then, 30 ml of 25% (w/w) aqueous solution of sulfuric acid are added. The reaction is stirred for 5-10 minutes. 50 ml of water and 120 ml of isopropyl acetate are added to the reaction mixture. The reaction mixture is stirred for 20 minutes at -5 - +10°C. The phases are separated and the organic phase is discarded. 1.5 g of ammonium sulfate is added to aqueous phase and stirred till dissolution. The aqueous phase is washed with 2 x 120 ml of isopropyl acetate at -5 - +10°C. The product is isavuconazonium bisulfate contained in the aqueous phase.

### Example 5b: Isolation of isavuconazonium sulfate

50 g of ammonium sulfate is added to the aqueous solution of Example 5a and stirred for 10 minutes at -5 - +10°C. 40 ml of THF are added and the obtained mixture is stirred for 20 minutes at -5 - +10°C. The phases are separated and the aqueous phase is discarded. The organic phase is washed twice with 65 ml of 40% (w/w) aqueous solution of ammonium sulfate. 6 ml of methanol, 48 ml of isopropanol and 6 g of anhydrous magnesium sulfate are added to the organic phase. The obtained suspension is stirred for 20 minutes at -5 - +10°C. Filtration is performed to remove inorganic solids. The filter is washed with a mixture of 9 ml of THF and 1 ml of methanol. In a second reactor 550 ml of methyl tert-butyl ether and 95 ml of isopropanol are cooled to 0 - 5°C. The filtrate obtained from isolation of inorganic solids is added dropwise to a mixture of methyl tert-butyl ether and isopropanol. The obtained suspension is stirred at 0 - 10°C for 30 minutes and filtered off. The product on the filter is washed with 2 x 60 ml of methyl tert-butyl ether and dried at RT (20 - 25°C) under vacuum. 25 g of isavuconazonium sulfate crude are obtained (containing about 10% of solvents, 0.027 mol, yield about 77%).

### Example 6a: Synthesis of isavuconazonium sulfate

ISV Br HBr obtained as in Example 3 is dissolved in 40 ml of DM water and 220 ml of isopropyl acetate at 0 - 5°C. A solution of 10 g (0.25 mol) of NaOH in 330 ml of water is prepared in a second reactor. 55 g (0.259 mol) of 2,3,4,5,6-pentafluorobenzoic acid are added to the NaOH solution, stirred till dissolution and cooled to 0 - 10°C. 2/3 of prepared sodium 2,3,4,5,6-pentafluorobenzoate are added to the ISV Br HBr solution and the obtained mixture is stirred for 20 minutes at -5 - +10°C. The phases are separated and the aqueous phase is discarded. The rest (1/3) of the sodium 2,3,4,5,6-pentafluorobenzoate solution is added to the organic phase and the obtained mixture is stirred for 20 minutes at -5 - +10°C. 40 ml of DM water and 140 ml of 20% (w/w) aqueous solution of NaHSO₄ are added to the organic phase. The reaction mixture is stirred for 20 minutes at -5 - +5°C. The phases are separated and the organic phase is discarded. The product is isavuconazonium bisulfate contained in the aqueous phase.

### Example 6b: Isolation of isavuconazonium sulfate

1.5 g of ammonium sulfate are added to the aqueous solution of Example 6a and stirred to dissolution. The aqueous phase is washed with 2 x 120 ml of isopropyl acetate at -5 - +10°C. 65 g of ammonium sulfate are added to the aqueous phase and stirred for 10 minutes at -5 - +10°C. 40 ml of THF are added and the obtained mixture is stirred for 20 minutes at -5 - +10°C. The phases are separated and the aqueous phase is discarded. The organic phase is washed twice with 65 ml of 40% (w/w) aqueous solution of ammonium sulfate. 6 ml of methanol, 48 ml of isopropanol and 6 g of anhydrous magnesium sulfate are added to the organic phase. The obtained suspension is stirred for 20 minutes at -5 - +10°C. Filtration is performed to remove inorganic solids. The filter is washed with a mixture of 9 ml of THF and 1 ml of methanol. In a second reactor 550 ml of methyl tert-butyl ether and 95 ml of isopropanol are cooled to 0 - 5°C. The filtrate obtained from isolation of inorganic solids is added dropwise to a mixture of methyl tert-butyl ether and isopropanol. The obtained suspension is stirred at 0 - 10°C for 30 minutes and filtered off. The product on the filter is washed with 2 x 60 ml of methyl tert-butyl ether and dried at RT (20 - 25°C) under vacuum. 25 g of isavuconazonium sulfate crude are obtained (containing about 10% of solvents, 0.027 mol, yield about 77%).

### Example 7a: Synthesis of isavuconazonium sulfate (variant 4 with 2,6-dihydroxybenzoic acid)

ISV Br HBr obtained as in Example 3 is dissolved in 40 ml of DM water and 220 ml of isopropyl acetate at 0 - 5°C. A solution of 10 g (0.25 mol) of NaOH in 330 ml of water is prepared in a second reactor. 40 g (0.259 mol) of 2,6-dihydroxybenzoic acid is added to the NaOH solution, stirred till dissolution and cooled to 0 - 10°C. 2/3 of prepared sodium 2,6-dihydroxybenzoate are added to the ISV Br HBr solution and the obtained mixture is stirred for 20 minutes at -5 - +5°C. The phases are separated and the aqueous phase is discarded. The rest (1/3) of the sodium 2,6-dihydroxybenzoate solution is added to the organic phase and the obtained mixture is stirred for 20 minutes at -5 - +10°C. 100 ml of DM water and 30 ml of 25% (w/w) aqueous solution of sulfuric acid are added to the organic phase. The reaction mixture is stirred for 20 minutes at -5 - +5°C. The phases are separated and the organic phase is discarded. Isavuconazonium bisulfate is obtained and is used in the next step with further workup.

### Example 7b: Isolation of isavuconazonium sulfate

1.5 g of ammonium sulfate are added to the aqueous solution of Example 7a and stirred to dissolution. The aqueous phase is washed with 2 x 120 ml of isopropyl acetate at -5 - +10°C. 65 g of ammonium sulfate are added to the aqueous phase and stirred for 10 minutes at -5 - +10°C. 40 ml of THF are added and the obtained mixture is stirred for 20 minutes at -5 - +10°C. The phases are separated and the aqueous phase is discarded. The organic phase is washed twice with 65 ml of 40% (w/w) aqueous solution of ammonium sulfate. 6 ml of methanol, 48 ml of isopropanol and 6 g of anhydrous magnesium sulfate are added to the organic phase. The obtained suspension is stirred for 20 minutes at -5 to +10°C. Filtration is performed to remove inorganic solids. The filter is washed with mixture of 9 ml of THF and 1 ml of methanol. In a second reactor 550 ml of methyl tert-butyl ether and 95 ml of isopropanol are cooled to 0 - 5°C. The filtrate obtained from isolation of inorganic solids is added dropwise to a mixture of methyl tert-butyl ether and isopropanol. The obtained suspension is stirred at 0 - 10°C for 30 minutes and filtered off. The product on the filter is washed with 2 x 60 ml of methyl tert-butyl ether and dried at RT (20 - 25°C) under vacuum. 18 g of isavuconazonium sulfate crude are obtained (containing about 10% of solvents, 0.019 mol, yield about 55%).

### Example 8: Purification of crude isavuconazonium sulfate

28 ml of acetone and 25 ml of methanol are placed in a 200 ml glass reactor and cooled to 0-5°C. 25 g of crude isavuconazonium sulfate are added and the mixture is stirred for 10-20 minutes and filtered off. The filter is washed with a mixture of 3 ml acetone and 2 ml of methanol. 250 ml of methyl tert-butyl ether and 250 ml of acetone are placed in a second reactor and cooled to 0 - 5°C. The filtrate is added dropwise to the mixture of methyl tert-butyl ether and acetone at 0 - 5°C and stirred for 10-30 minutes. The precipitated product is filtered off and washed 2 x 60 ml of cyclohexane and dried at RT (20 - 25°C) under vacuum. 22 g of purified isavuconazonium sulfate are obtained (containing about 10% of solvents, 0.023 mol, yield 88%).

### Example 9: Drying of isavuconazonium sulfate

66 ml of water DM is cooled to 0 - 5°C. 22 g of isavuconazonium sulfate are added and the mixture is stirred for 10-20 minutes at 0 - 5°C. Reaction is left without stirring for 1 hour. Lower aqueous phase is freeze-dried (lyophilized) with a yield of about 19.3 g isavuconazonium sulfate (yield 88%, HPLC purity 98.8%).

## Claims

1. A process for the preparation of isavuconazonium sulfate comprising a1) contacting the salt of Formula 1 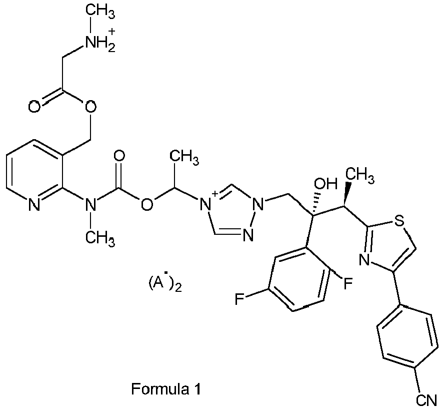 in a system of at least one organic solvent and water with a source of bisulfate anions,
wherein A is an anion of a lipophilic organic acid HA.

2. The process according to claim 1, wherein the lipophilic organic acid HA has a pKa in water between -2 and 4.

3. The process according to claim 1 or 2, wherein the lipophilic organic acid HA is selected from aromatic carboxylic acids, preferably aromatic monocarboxylic acids.

4. The process according to any one of claims any one of the preceding claims, wherein the lipophilic organic acid HA is selected from benzoic acid and naphthalenic acid, wherein the aromatic moiety is unsubstituted or is substituted with one or more groups selected from hydroxyl, (C₁-C₆)-alkyl-(C=O)-, halogen, (C₁-C₆)-alkoxyl, nitro, cyano, (C₁-C₆)-alkyl.

5. The process according to any one of the preceding claims, wherein the lipophilic organic acid is selected from 2,3,4,5,6-pentafluorobenzoic acid or 2,6-dihydroxybenzoic acid, preferably the lipophilic organic acid is 2,3,4,5,6-pentafluorobenzoic acid.

6. The process according to any one of the preceding claims, wherein the at least one organic solvent is selected from at least one water miscible solvent, at least one water immiscible solvent, and a mixture thereof, wherein the at least one water miscible solvent is selected from ketones, for example acetone; alcohols, for example methanol, ethanol, isopropanol, n-propanol; ethers, for example THF, 1,4-dioxane; acetonitrile or acetic acid; and the at least one water immiscible solvent is selected from esters of acetic acid, for example ethyl acetate, isopropyl acetate; ethers, for example diethyl ether, diisopropyl ether, methyl tert-butyl ether; halogenated (C₁-C₂)-alkanes, for example dichloromethane, dichloroethane, chloroform; aromatic solvents, for example toluene, xylenes.

7. The process according to any one of the preceding claims wherein the source of bisulfate anions is sulfuric acid or a bisulfate salt, wherein the bisulfate salt is preferably selected from alkali metal, alkaline earth metal and ammonium bisulfates.

8. The process according to any one of the preceding claims, wherein the process for obtaining the salt of Formula 1 comprises
a0) contacting the compound of Formula 2 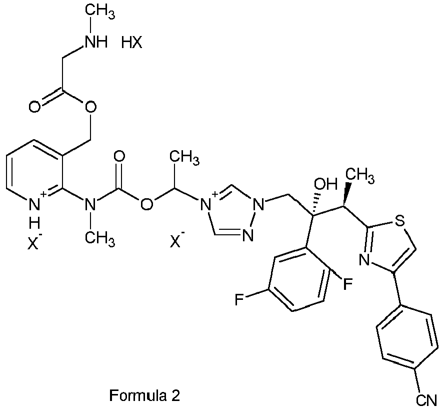 with a salt of a lipophilic organic acid HA,
wherein X is a halogen atom X selected from Cl, Br and I.

9. Isavuconazole hydrobromide THF solvate.

10. A process for preparing, isolating and/or purifying isavuconazonium sulfate comprising the following steps:
(i) providing an aqueous solution of isavuconazonium bisulfate and/or sulfate,
(ii) adding an alkali metal sulfate or ammonium sulfate and an organic solvent,
(iii) separating the organic phase,
(iv) combining the obtained organic phase with an antisolvent or a mixture of antisolvents to precipitate isavuconazonium sulfate.

11. The process according to claim 10, wherein the organic solvent of step (ii) is selected from ethers, for example THF, 2-methyltetrahydrofurane; esters of acetic acid, for example isopropyl acetate; ketones, for example acetone, methyl ethyl ketone; alcohols, for example methanol, isopropanol; halogenated (C₁-C₂)-alkanes, for example dichloromethane; acetonitrile or acetic acid; and mixtures thereof.

12. A salt of isavuconazonium with a lipophilic organic acid HA.

13. The salt of isavuconazonium according to claim 12, wherein the lipophilic organic acid HA has a pKa in water between -2 and 4 and wherein it is preferably selected from aromatic carboxylic acids, more preferably aromatic monocarboxylic acids.

14. The salt of isavuconazonium according to claim 12 or 13, wherein the lipophilic organic acid HA is selected from benzoic acid and naphthalenic acid, wherein the aromatic moiety is unsubstituted or is substituted with one or more groups selected from hydroxyl, (C₁-C₆)-alkyl-(C=O)-, halogen, (C₁-C₆)-alkoxyl, nitro, cyano, (C₁-C₆)-alkyl.

15. A salt of isavuconazonium according to any one of the claims 12-14, wherein the lipophilic organic acid is 2,3,4,5,6-pentafluorobenzoic acid or 2,6-dihydroxybenzoic acid.

16. A use of a salt of any one of claims 12-15 in the preparation of isavuconazonium sulfate.
